(19) Europäisches Patentamt
European Patent Office
Office européen des brevets

(11) Veröffentlichungsnummer: **0 301 414 B1**

# (12) EUROPÄISCHE PATENTSCHRIFT

(45) Veröffentlichungstag der Patentschrift: **03.03.93**

(51) Int. Cl.5: **C07C 303/02**, C07C 309/08, C07C 303/32, C11D 1/16

(21) Anmeldenummer: **88111771.7**

(22) Anmeldetag: **21.07.88**

## (54) Oberflächenaktive Hydroxysulfonate.

(30) Priorität: **29.07.87 DE 3725030**

(43) Veröffentlichungstag der Anmeldung:
**01.02.89 Patentblatt 89/05**

(45) Bekanntmachung des Hinweises auf die Patenterteilung:
**03.03.93 Patentblatt 93/09**

(84) Benannte Vertragsstaaten:
**DE FR GB**

(56) Entgegenhaltungen:
**EP-A- 0 137 983**
**CH-A- 162 732**

(73) Patentinhaber: **Henkel Kommanditgesellschaft auf Aktien**
**Postfach 1100 Henkelstrasse 67**
**W-4000 Düsseldorf-Holthausen(DE)**

(72) Erfinder: **Fabry, Bernd, Dr.**
**Danziger Strasse 31**
**W-4052 Korschenbroich(DE)**
Erfinder: **Piorr, Robert, Dr.**
**Kieselei 12**
**W-4030 Ratingen-Hösel(DE)**
Erfinder: **Schumacher, Astrid**
**Gleiwitzerstrasse 6**
**W-4000 Düsseldorf 1(DE)**

EP 0 301 414 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung wässriger Lösungen von Alkali-, Erdalkali- oder Ammoniumsalzen oberflächenaktiver Hydroxysulfonate aus ungesättigten Fettalkoholen mit 16 - 22 C-Atomen und deren Oxalkylaten durch Sulfonierung von Estern niederer Alkansäuren dieser Alkohole und Oxethylate und Hydrolyse der Sulton- und Estergruppen.

Ungesättigte Fettalkohole und deren Oxalkylierungsprodukte werden bei der Umsetzung mit Schwefeltrioxid sowohl an der Doppelbindung sulfoniert als auch an der Hydroxylgruppe sulfatiert. Da Sulfonattenside eine hohe Stabilität gegen Hydrolyse aufweisen, während Sulfattenside in saurem wässrigem Medium leicht hydrolytisch gespalten werden, bestand die Aufgabe, aus ungesättigten Fettalkoholen und deren Oxalkylaten einheitliche, von Sulfatestergruppen freie und gegen Hydrolyse stabile Tenside herzustellen.

Es war bereits in DE-A-3 331 513 vorgeschlagen worden, aus den Niedrigalkylethern ungesättigter Fettalkohole und Fettalkoholpolyalkylenglycolether durch Sulfonierung hydrolysestabile Ethersulfonate herzustellen. Nunmehr wurde ein noch einfacheres Verfahren zur Herstellung von Sulfonattensiden aus ungesättigten Fettalkoholen gefunden:
Gegenstand der Erfindung ist ein Verfahren zur Herstellung wäßriger Lösungen von Alkali-, Erdalkali- oder Ammoniumsalzen oberflächenaktiver Hydroxysulfonate, dadurch gekennzeichnet, daß man einen ungesättigten Fettalkyl- oder Fettalkylpolyoxyalkylester der Formel (I)

$$R^1-O-(C_nH_{2n}O)_x-\overset{\overset{\displaystyle O}{\parallel}}{C}-R^2$$

in welcher $R^1$ eine lineare Alkenylgruppe mit 16 - 22 C-Atomen oder eine überwiegend aus Oleyl-, Palmitoleyl-, Linoleyl, Gadoleyl- und/oder Erucylgruppen bestehende Fettalkylgruppe, n eine ganze Zahl von 2 bis 4, x = 0 oder eine Zahl bis 30 und $R^2CO$ eine Acylgruppe mit 1 bis 4 C-Atomen darstellt, mit Schwefeltrioxid umsetzt, das Umsetzungsprodukt in die wäßrige Lösung von 1 - 2,5 Mol Alkali-, Erdalkali- oder Ammoniumhydroxid pro Mol angelagertes $SO_3$ einträgt und die Lösung bis zur Hydrolyse der enthaltenen Ester- und Sultongruppen erwärmt.

Hydroxysulfonate mit besonders günstigen oberflächenaktiven Eigenschaften werden erhalten, wenn zur Sulfonierung ein ungesättigter Fettalkohol- oder Fettalkylpolyoxyalkyl-ester der Formel (I), in der n = 2 und x = 0 oder eine Zahl bis 10 ist, d. h. ein Ester eines ungesättigten Fettalkohols oder eines Anlagerungsprodukts von bis zu 10 Mol Ethylenoxid an einen ungesättigten Fettalkohol eingesetzt wird. Die Gruppe $R^2$-CO kann eine Formyl-, Acetyl-, Propionyl oder Butyrylgruppe sein; bevorzugt ist die Acetylgruppe. Die Gruppe $R^1$ ist bevorzugt eine Oleylgruppe oder ein überwiegend aus Oleylgruppen bestehender Fettalkylrest.

Die Sulfonierung der ungesättigten Fettalkyl- oder Fettalkyl-polyoxyalkyl-ester der Formel (I) wird bevorzugt mit gasförmigem Schwefeltrioxid bei Temperaturen von 10 - 80° C durchgeführt. Bevorzugt wird die Sulfonierung bei niedrigen Temperaturen von 20 - 50° C mit einem Gemisch aus $SO_3$ und Luft oder Inertgas, z. B. Stickstoff, mit vorzugsweise 1 - 10 Vol. % $SO_3$, durchgeführt. Die Umsetzung mit Schwefeltrioxid (Sulfonierung) läßt sich in üblichen, für die Sulfatierung von Fettalkoholen oder die Sulfonierung von Fettsäureestern, Alkylbenzol oder Olefinen geeigneten und gebräuchlichen Reaktoren, bevorzugt vom Typ der Fallfilmreaktoren, kontinuierlich durchführen.

Das rohe Sulfonierungsprodukt wird in eine wässrige Lösung eines Alkali- oder Erdalkalihydroxids oder von Ammoniumhydroxid eingetragen, wobei dieses in einer Menge von 1 - 2,5 Mol pro Mol angelagerten Schwefeltrioxids vorliegen sollte. Das Alkalihydroxid dient der Neutralisation des Sulfonierungsproduktes und der Überschuß an Alkali ist erforderlich, um das im Sulfonierungsprodukt gelöste, gasförmige $SO_3$ zu neutralisieren und einen Alkaliüberschuß aufrechtzuerhalten, der die anschließende Hydrolysestufe katalysiert. Als Neutralisationsbase wird bevorzugt Natriumhydroxid eingesetzt.

Die Konzentration der Neutralisationsbase in Wasser wird bevorzugt so gewählt, daß das Endprodukt eine noch fließfähige oder pumpbare Lösung bildet.

Das Sulfonierungsprodukt enthält neben ungesättigten Estersulfonsäuren vor allem Sultone. Unter den Bedingungen der Sulfonierung kommt es zu einer Isomerisierung der ungesättigten Fettalkyl- oder Fettalkylpolyoxyalkylester der Formel (I), wobei die Position der olefinischen Doppelbindungen sich statistisch über die Alkenylgruppe verteilt. Bei der Reaktion des $SO_3$ mit der olefinischen Doppelbindung entstehen wahrscheinlich zunächst 1,2-Sultone, die sehr schnell zu 1,3-Sultonen und langsamer zu 1,4-Sultonen, bei höherem Temperaturen auch zu ungesättigten Sulfonsäuren isomerisieren.

Zur Überführung der bei der Sulfonierung primär gebildeten Sultone in Hydroxysulfonate ist es erforderlich, die wäßrige Lösung einer Hydrolysestufe zu unterwerfen. Die Hydrolyse wird durch Erwärmen der Lösung bis zur völligen Zerstörung der enthaltenen Sultongruppen durchgeführt. Dabei werden auch die enthaltenen Estergruppen hydrolysiert. Die für die Hydrolyse der Ester- und Sultongruppen erforderliche Zeit ist von den Hydrolysebedingungen abhängig. So läßt sich z. B. bei Siedetemperatur unter Normaldruck in 4 Stunden, bei höherer Temperatur unter Druck aber in erheblich kürzerer Zeit eine vollständige Hydrolyse zum Hydroxysulfonat erreichen.

Die nach dem erfindungsgemäßen Verfahren erhältlichen Gemische oberflächenaktiver Hydroxysulfonate sind daher ganz oder überwiegend aus Verbindungen der Formeln (II) oder (III)

$$(II)\quad CH_3-(CH_2)_y-\underset{\large OH}{\underset{|}{CH}}-(CH_2)_p-\underset{\large SO_3H}{\underset{|}{CH}}-(CH_2)_z-CH_2-O-(C_nH_{2n}O)_x-H$$

$$(III)\quad CH_3-(CH_2)_y-\underset{\large SO_3H}{\underset{|}{CH}}-(CH_2)_p-\underset{\large OH}{\underset{|}{CH}}-(CH_2)_z-CH_2-O-(C_nH_{2n}O)_x-H$$

oder deren Alkali-, Erdalkali- oder Ammoniumsalzen zusammengesetzt, wobei y und z = 0 oder Zahlen von 1 bis 18, p = 0, 1 oder 2 und die Summe (y + z + p) eine Zahl von 12 bis 18, x = 0 oder eine Zahl bis 30 und n eine ganze Zahl von 2 bis 4 sein kann. Solche Gemische oberflächenaktiver Hydroxysulfonate sind daher ebenfalls Gegenstand der Erfindung.

Die oberflächenaktiven Hydroxysulfonate, wie sie nach dem erfindungsgemäßen Verfahren erhalten werden, fallen in Form von dunkel- bis hellgelben, wäßrigen, alkalischen Lösungen der Alkalisalze an. Diese könne, wenn dies gewünscht wird, mit Wasserstoffperoxidlösung oder Alkalihypochlorit-Lösung ("Chlorlauge") in bekannter Weise gebleicht werden. Der pH-Wert der Lösungen kann unter Verwendung von z. B. Salzsäure, Schwefelsäure, Phosphorsäure, Citronensäure oder Milchsäure neutral eingestellt werden. Zur Stabilisierung gegen Bakterienbefall empfiehlt sich eine Konservierung, z.B. mit Formaldehydlösung, p-Hydroxybenzoat, Sorbinsäure oder anderen bekannten Konservierungsstoffen.

Die als Ausgangsprodukte zu verwendenden Fettalkyl- oder Fettalkyl-polyoxyalkyl-ester der Formel (I) sind nach literaturbekannten Verfahren zugänglich: Ihre Herstellung geht aus von ungesättigten Fettalkoholen, z. B. von Oleylalkohol oder technischen, überwiegend aus Oleylalkohol, Palmitoleylalkohol, Linoleylalkohol, Gadoleylalkohol oder Erucylalkohol bestehenden Alkoholschnitten. Geringe Anteile an gesättigten Alkoholen, z.B. an Cetyl- und Stearylalkohol sind tragbar, vor allem wenn die daraus durch Oxalkylierung hergestellten Produkte selbst wasserlöslich sind. Geeignete ungesättigte Alkohole sind durch Hydrierung von Ölsäure oder von technischen Oleinsäuren herstellbar und im Handel erhältlich. Bevorzugt werden technische Cetyl-Oleyl- und Oleyl-Linoleyl-alkoholschnitte mit einer Jodzahl im Bereich von 70 bis 130 eingesetzt.

Die Oxalkylierung ungesättigter Alkohole mit Ethylenoxid, Propylenoxid, Butylenoxid oder Gemischen dieser Alkylenoxide ist ein bekanntes großtechnisches Verfahren. Dabei werden Gemische homologer Oxalkylate erhalten, deren mittlerer Oxalkylierungsgrad x der molaren Menge des angelagerten Alkenyloxids entspricht.

Bevorzugt werden die nicht oxalkylierten ungesättigten Alkohole oder die Anlagerungsprodukte von bis zu 10 Mol Ethylenoxid verwendet.

Die Veresterung der endständigen Hydroxylgruppe der ungesättigten Alkohole und/oder deren Oxalkylate erfolgt nach ebenfalls literaturbekannten Methoden. Sie kann z. B. durch Umsetzung mit einer Carbonsäure der Formel $R^2$-COOH in Gegenwart eines Katalysators, z.B. konzentrierter Schwefelsäure (ca. 10 ml $H_2SO_4$ pro Mol Alkohol) oder Zinnschliff (ca. 0,05 bis 0,1 Mol pro Mol Alkohol) in der Siedehitze unter Abscheidung des Reaktionswassers erfolgen. Die Veresterung kann aber auch durch Umsetzung mit einem Carbonsäureanhydrid der Formel $R^2COOCOR^2$ unter bekannten Bedingungen durchgeführt werden. Es empfiehlt sich, für die Veresterung die Carbonsäure oder das Carbonsäureanhydrid in einem molaren Überschuß von ca. 10 bis 20 Mol% einzusetzen. Die Veresterung mit einem Carbonsäureanhydrid ist in der Regel in einer Zeit von 2 bis 4 Stunden, etwa bei einer Temperatur die der Siedetemperatur der Carbonsäure entspricht, weitgehend abgeschlossen.

Die nach dem erfindungsgemäßen Verfahren zugänglichen Hydroxysulfonate weisen eine hohe Oberflächenaktivität und gute anwendungstechnische Tensideigenschaften auf. Besonders günstig ist das Schäumvermögen der Alkali- und Ammoniumsalze, welches das von Alphasulfofettsäureestern und Alkylbenzolsulfonaten zum Teil deutlich übertrifft. Die Erdalkalisalze sind relativ schaumarme Tenside. Auch das textile Netzvermögen ist stark ausgeprägt, so daß die Produkte sowohl als technische Netzmittel als auch für die Anwendung in Wasch- und Reinigungsmitteln geeignet erscheinen. Besonders hervorzuheben ist die Hydrolysestabilität, auch im sauren Medium, was den Anwedungsbereich z. B. gegenüber Fettalkoholsulfaten und Alkylethersulfaten erheblich ausweitet.

Die folgenden Beispiele sollen den Erfindungsgegenstand näher erläutern:

## Beispiele

1.1 Oleylacetat (technisch)

Ein technischer Oleylalkohol (HD-Ocenol(R)90/95, Jodzahl 94, Hydroxylzahl 210) wurde mit Essigsäureanhydrid (20 Mol% Überschuß) bei 118 °C 4 Stunden umgesetzt. Das Reaktionsgemisch wurde dann auf Eiswasser gegossen und die organische Phase mehrmals mit Wasser gewaschen. Dann wurde der erhaltene Rohester getrocknet und durch Destillation gereinigt. Der erhaltene Ester hatte eine Jodzahl von 83, eine Resthydroxylzahl von 0,9.

1.2 Herstellung des Hydroxysulfonats

1.2.1 Sulfonierung mit 1 Mol $SO_3$

In einem 800-ml-Standreaktor mit Mantelkühlung wurden 310 g (1 Mol) des Oleylacetats aus 1.1 vorgelegt und bei 30 °C mit 80 g (1 Mol) $SO_3$ sulfoniert. Das $SO_3$ wurde durch Erhitzen aus einer entsprechenden Menge Oleum ausgetrieben, mit $N_2$ auf eine Konzentration von 5 Vol% verdünnt und innerhalb von 32 Minuten in das Oleylacetat eingeleitet, wobei die Temperatur des Reaktionsgemisches durch Kühlung auf Werte unterhalb 40 °C gehalten wurde.

Nach der Sulfonierung wurde das Reaktionsgemisch auf 10 °C abgekühlt und in eine verdünnte Lösung von 84 g (2,1 Mol) NaOH in 1 500 g Wasser eingerührt. Dieses Gemisch wurde dann 4 Stunden auf dem Dampfbad bei 95 bis 100 °C hydrolysiert. Nach dem Abkühlen auf 20 °C wurde durch Zugabe von HCl-Lösung der pH-Wert des Reaktionsgemisches auf 7,0 eingestellt.

Das erhaltene Produkt hatte folgende Kennzahlen:

| | |
|---|---|
| Aniontensid (Zweiphasentitrationsmethode nach Einheitsmethode DGF-H-III-10): | 0,517 mval/g |
| Unsulfierte Anteile (DGF-G-III-6b): | 4,0 Gew.-% |
| $Na_2SO_4$: | 2,0 Gew.-% |
| $CH_3COONa$: | 6,0 Gew.-% |
| Klett-Farbzahl (1-cm-Küvette): | 82 |
| (nach 30 Minuten Bleichung mit 2%iger wäßriger Lösung von Natriumhypochlorit) | |

1.2.2 Sulfonierung mit 1,3 Mol $SO_3$

Versuch 1.2.1 wurde wiederholt, wobei 1,3 Mol $SO_3$ (104 g) innerhalb 30 Minuten in das Oleylacetat eingeleitet wurden. Das erhaltene Hydroxysulfonat hatte die folgenden Kennzahlen:

| | |
|---|---|
| Aniontensid (DGF-H-III-10): | 0,594 mval/g |
| Unsulfierte Anteile (DGF-G-III-6b): | 2,0 Gew.-% |
| $Na_2SO_4$: | 2,0 Gew.-% |
| $CH_3COONa$: | 6,0 Gew.-% |
| Klett-Farbzahl: | 280 |
| (Die Messung der Klett-Farbzahl erfolgte bei einer Konzentration von 5 Gew.-% Aniontensid, bei pH = 7 unter Verwendung einer 1-cm-Küvette und eines Blaufilters (400 bis 465 mμ)) | |

1.2.3 Sulfonierung mit 1,8 Mol $SO_3$

Versuch 1.2.1 wurde wiederholt, wobei 1,8 Mol $SO_3$ (144 g) innerhalb 40 Minuten in das Oleylacetat eingeleitet wurden. Das erhaltene Hydroxysulfonat hatte die folgenden Kennzahlen:

| | |
|---|---|
| Aniontensid (DGF-H-III-10): | 0,723 mval/g |
| Unsulfierte Anteile (DGF-G-III-6b): | 2,0 Gew.-% |
| $Na_2SO_4$: | 2,0 Gew.-% |
| $CH_3COONa$: | 4,0 Gew.-% |
| Klett-Farbzahl: | 425 |

1.2.4 Sulfonierung gemäß 1.2.1 bei 50 bis 60 °C

Versuch 1.2.1 wurde wiederholt, wobei die Temperatur des Reaktionsgemisches während der $SO_3$-Einleitung in einem Bereich zwischen 50 und 60 °C gehalten wurde. Das dabei erhaltene Hydroxysulfonat hatte die folgenden Kennzahlen:

| | |
|---|---|
| Aniontensid (DGF-H-III-10): | 0,516 mval/g |
| Unsulfierte Anteile (DGF-G-III-6b): | 4,0 Gew.-% |
| $Na_2SO_4$: | 5,0 Gew.-% |
| $CH_3COONa$: | 4,0 Gew.-% |
| Klett-Farbzahl: | 140 |

1.2.5 Herstellung im Fallfilmreaktor

In einem kontinuierlich arbeitenden Fallfilmreaktor wurden 3,1 kg (10 Mol) Oleylacetat (nach 1.1) bei 30 °C mit einem Durchsatz von 10 g/min mit $SO_3$ im Molverhältnis Oleylacetat : $SO_3$ = 1 : 1,3 zur Reaktion gebracht. Das rohe Sulfonierungsprodukt wurde kontinuierlich in verdünnte wäßrige Natronlauge eingerührt und dann wie unter 1.2.1 hydrolysiert und aufgearbeitet. Das erhaltene Produkt hatte folgende Kennzahlen:

| | |
|---|---|
| Aniontensid (DGF-H-III-10): | 1,498 mval/g |
| Unsulfierte Anteile (DGF-G-III-6b): | 5,0 Gew.-% |
| $Na_2SO_4$: | 1,0 Gew.-% |
| $CH_3COONa$: | 5,0 Gew.-% |
| Klett-Farbzahl: | 48 |
| (nach Bleichung mit 5 % $H_2O_2$) | |

1.2.6 Neutralisation mit Calciumhydroxid

Versuch 1.2.2 wurde wiederholt, wobei das Reaktionsgemisch nach der Sulfonierung abgekühlt und in eine verdünnte Lösung von 155 g (2,1 Mol) $Ca(OH)_2$ in 1 500 g Wasser eingerührt wurde. Das Gemisch wurde dann 8 Stunden auf dem Dampfbad bei 95 bis 100 °C hydrolysiert und das ausgefallene unlösliche Calciumsulfat abfiltriert. Nach dem Abkühlen auf 20 °C wurde durch Zugabe von verdünnter Salzsäure der pH-Wert des Reaktionsgemisches auf 7,0 eingestellt. Das Produkt hatte folgende Kennzahlen:

| | |
|---|---|
| Aniontensid (DGF-H-III-10): | 0,521 mval/g |
| Unsulfierte Anteile (DGF-G-III-6b): | 4 Gew.-% |
| $CaSO_4$: | 0 Gew.-% |
| $(CH_3COO)_2Ca$: | 6 Gew.-% |
| Klett-Farbzahl: | 200 |

1.2.7 Neutralisation mit Ammoniak

Versuch 1.2.2 wurde wiederholt, wobei das Reaktionsgemisch nach der Sulfonierung abgekühlt und in eine verdünnte Lösung von 36 g $NH_3$ (2,1 Mol) in 1 500 g Wasser eingerührt wurde. Das Gemisch wurde 8 Stunden auf dem Dampfbad bei 95 bis 100 °C hydrolysiert. Nach dem Abkühlen auf 20 °C wurde durch Zugabe von verdünnter Salzsäure der pH-Wert des Reaktionsgemisches auf 7,0 eingestellt. Das Produkt hatte folgende Kennzahlen:

| | |
|---|---|
| Aniontensid (DGF-H-III-10): | 0,419 mval/g |
| Unsulfierte Anteile (DGF-G-III-6b): | 5 Gew.-% |
| $(NH_4)_2SO_4$: | 4 Gew.-% |
| $CH_3COONH_4$: | 1 Gew.-% |
| Klett-Farbzahl: | 140 |

2.1 Oleyl-oxyethyl(1 EO)-acetat

Ein technischer Oleylalkohol (gemäß Beispiel 1.1) wurde nach bekanntem Verfahren (Na-methylat als Katalysator, 170 °C) mit 1 Mol Ethylenoxid pro Mol Oleylalkohol oxethyliert. Das Oxethylat wurde analog Beispiel 1.1 in das Acetat überführt.

2.2 Herstellung des Hydroxysulfonats

Ausgehend von 230 g des Produkts nach Beispiel 2.1 wurde durch Sulfonierung analog Beispiel 1.2.1 mit 1,2 Mol $SO_3$ pro Mol Oleyl-oxyethyl-acetat, die innerhalb 12 Minuten eingeleitet wurden und Aufarbeitung gemäß Beispiel 1.2.1 ein Hydroxysulfonat mit den folgenden Kennzahlen erhalten:

| | |
|---|---|
| Aniontensid (DGF-H-III-10): | 0,487 mval/g |
| Unsulfierte Anteile (DGF-G-III-6b): | 3,0 Gew.-% |
| $Na_2SO_4$: | 3,0 Gew.-% |
| $CH_3COONa$: | 4,0 Gew.-% |
| Klett-Farbzahl: | 114 |

3.1 Oleyl-polyoxyethyl(5 EO)-acetat

Analog Beispiel 2.1 wurden an einen technischen Oleylalkohol (gemäß Beispiel 1.1) 5 Mol Ethylenoxid angelagert. Das Oxethylat wurde dann, ebenfalls analog Beispiel 1.1 in das Acetat überführt.

3.2 Herstellung des Hydroxysulfonats

Ausgehend von 320 g des Oleyl-polyoxyethyl(5 EO)-acetats nach Beispiel 3.1 wurde durch Sulfonierung analog Beispiel 1.2.1 mit 1,3 Mol $SO_3$ pro Mol des Oleyl-polyoxyethyl(5 EO)-acetats, die innerhalb von 11 Minuten eingeleitet wurden und Aufarbeitung gemäß Beispiel 1.2.1 ein Hydroxysulfonat mit den folgenden Kennzahlen erhalten:

| | |
|---|---|
| Aniontensid (DGF-H-III-10): | 0,362 mval/g |
| Unsulfierte Anteile (DGF-G-III-6b): | 6,0 Gew.-% |
| $Na_2SO_4$: | 3,0 Gew.-% |
| $CH_3COONa$: | 3,0 Gew.-% |
| Klett-Farbzahl: | 21 |

4.1 Oleyl-polyoxyethyl(10 EO)-acetat

Analog Beispiel 2.1 wurde an einen technischen Oleylalkohol (gemäß Beispiel 1.1) 10 Mol Ethylenoxid angelagert. Das Oxethylat wurde dann, ebenfalls analog Beispiel 1.1 in das Acetat überführt.

4.2 Herstellung des Hydroxysulfonats

Ausgehend von 540 g des Oleyl-polyoxyethyl(10 EO)-acetats nach Beispiel 4.1 wurde durch Sulfonierung analog Beispiel 1.2.1 mit 1,3 Mol $SO_3$ pro Mol Oleyl-polyoxyethyl(10 EO)-acetat, die innerhalb von 15 Minuten eingeleitet wurden und Aufarbeitung analog Beispiel 1.2.1 ein Hydroxysulfonat mit den folgenden Kennzahlen hergestellt:

| | |
|---|---|
| Aniontensid (DGF-H-III-10): | 0,242 mval/g |
| Unsulfierte Anteile (DGF-G-III-6b): | 6,0 Gew.-% |
| $Na_2SO_4$: | 3,0 Gew.-% |
| $CH_3COONa$: | 3,0 Gew.-% |
| Klett-Farbzahl: | 19 |

5. Anwendungstechnische Prüfungen

Die Hydroxysulfonate der Beispiele 1 bis 4 wurden anwendungstechnisch bezüglich der Schäumeigenschaften und des Netzvermögens geprüft.

Schäumvermögen

Das Schäumvermögen wurde in der Weise bestimmt, daß 100 ml einer Lösung von 1 g/l des Aniontensids in einem 250-ml-Schüttelzylinder unter definierten Bedingungen geschüttelt wurden. Es wurde das Schaumvolumen über der Lösung nach 0, 1, 3 und 5 Minuten nach dem Schütteln abgelesen. Die Bestimmung erfolgte bei 20 °C in entsalztem Wasser (0 °dH).

Netzvermögen

Das Netzvermögen wurde nach DIN 53901 "Bestimmung des Netzvermögens nach der Tauchnetzmethode" bestimmt. Es kamen Lösungen von 1 g/l Aniontensid bei 20 °C in entsalztem Wasser (0 °dH) zur Anwendung. Als Maß für die Netzwirkung dient die Zeitspanne, die ein genormtes Stoffläppchen benötigt, um nach dem Eintauchen in die Netzmittellösung vollständig benetzt zu werden und abzusinken.

In der folgenden Tabelle sind die Ergebnisse der Schäum- und Netzversuche als Mittelwerte von jeweils fünf Bestimmungen dargestellt:

| Produkt aus Beispiel | Schaumvolumen (ml) 0 Min. | 1 Min. | 3 Min. | 5 Min. | Netzzeit (s) |
|---|---|---|---|---|---|
| 1.2.1 | 300 | 170 | 60 | 18 | 91 |
| 1.2.2 | 300 | 180 | 100 | 20 | 28 |
| 1.2.3 | 200 | 145 | 10 | 0 | 93 |
| 1.2.4 | 190 | 130 | 10 | 0 | 180 |
| 1.2.5 | 300 | 170 | 60 | 25 | 32 |
| 1.2.6 | 10 | 10 | 8 | 8 | 300 |
| 1.2.7 | 230 | 170 | 110 | 40 | 13 |
| 2.2 | 300 | 200 | 150 | 75 | 220 |
| 3.2 | 300 | 200 | 190 | 150 | 261 |
| 4.2 | 300 | 200 | 150 | 100 | 300 |

## Patentansprüche

1. Verfahren zur Herstellung wäßriger Lösungen von Alkali-, Erdalkali- oder Ammoniumsalzen, von oberflächenaktiven Hydroxysulfonaten, dadurch gekennzeichnet, daß man einen ungesättigten Fettalkyl- oder Fettalkylpolyoxyalkyl-ester der Formel (I)

$$R^1\text{-O-}(C_nH_{2n}O)_x\text{-}\overset{O}{\overset{\|}{C}}\text{-}R^2$$

in welcher $R^1$ eine lineare Alkenylgruppe mit 16 - 22 C-Atomen oder eine überwiegend aus Oleyl-, Palmitoleyl-, Linoleyl-, Gadoleyl- und/oder Erucylgruppen bestehende Fettalkylgruppe, n eine Zahl von 2 bis 4, x = 0 oder eine Zahl bis 30 und $R^2CO$ eine Acylgruppe mit 1 bis 4 C-Atomen darstellt, mit Schwefeltrioxid umsetzt, das Umsetzungsprodukt in die wäßrige Lösung von 1 bis 2,5 Mol Alkali-, Erdalkali- oder Ammoniumhydroxid pro Mol angelagertes $SO_3$ einträgt und die Lösung bis zur Hydrolyse der enthaltenen Ester- und Sultongruppen erwärmt.

2. Verfahren nach Anspruch 1, dadurch gekennzeichnet, daß n = 2 und x = 0 oder eine Zahl bis 10 ist.

3. Verfahren nach den Ansprüchen 1 und 2, dadurch gekennzeichnet, daß $R^2CO$ eine Acetylgruppe ist.

4. Verfahren nach den Ansprüchen 1 bis 3, dadurch gekennzeichnet, daß die Umsetzung mit Schwefeltrioxid bei Temperaturen von 20 bis 50 °C mit einem Gemisch aus $SO_3$ und Luft oder Inertgas mit einem Gehalt von 1 bis 10 Vol% $SO_3$ durchgeführt wird.

5. Verfahren nach den Ansprüchen 1 bis 4, dadurch gekennzeichnet, daß $R^1$ eine Oleylgruppe oder ein überwiegend aus Oleylgruppen bestehender Fettalkylrest ist.

6. Oberflächenaktive Hydroxysulfonate und deren Alkali-, Erdalkali- und Ammoniumsalze wie sie erhalten werden nach dem Verfahren gemäß Anspruch 1 bis 5.

7. Gemisch oberflächenaktiver Hydroxysulfonate, das ganz oder überwiegend aus Verbindungen der Formeln (II) oder (III)

(II) 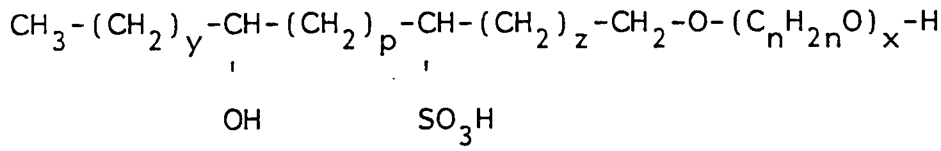

(III) 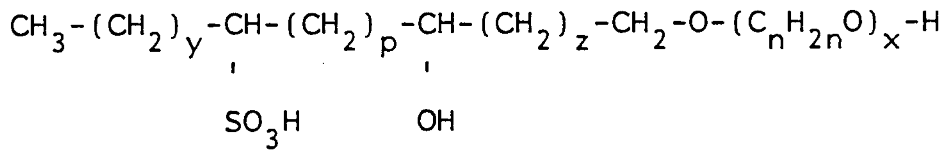

oder deren Alkali-, Erdalkali- und Ammoniumsalzen zusammengesetzt ist, wobei y und z = 0 oder Zahlen von 1 bis 18, p = 0, 1 oder 2 und die Summe (y+z+p) eine Zahl von 12 bis 18 und x = 0 oder eine Zahl bis 30 und n eine ganze Zahl von 2 bis 4 sein kann.

8. Verwendung von wäßrigen Lösungen der Alkali-, Erdalkali- und Ammoniumsalze oberflächenaktiver Hydroxysulfonate gemäß Anspruch 6 oder 7 zur Herstellung von Wasch- und Reinigungsmitteln.

**Claims**

1. A process for the production of aqueous solutions of alkali metal, alkaline-earth metal or ammonium salts of surface-active hydroxysulfonates, characterized in that an unsaturated fatty alkyl or fatty alkyl polyoxyalkyl ester corresponding to formula (I)

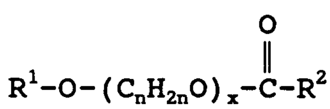

in which $R^1$ is a linear $C_{16-22}$ alkenyl group or a fatty alkyl group consisting predominantly of oleyl, palmitoleyl, linoleyl, gadoleyl and/or erucyl groups, n is a number of 2 to 4, x = 0 or a number of up to 30 and $R^2CO$ is a $C_{1-4}$ acyl group,
is reacted with sulfur trioxide, the reaction product is introduced into an aqueous solution of 1 to 2.5 mol alkali metal, alkaline-earth metal or ammonium hydroxide per mol added $SO_3$ and the solution is heated until the ester and sultone groups present have been hydrolyzed.

2. A process as claimed in claim 1, characterized in that n = 2 and x = 0 or a number of up to 10.

3. A process as claimed in claims 1 and 2, characterized in that $R^2CO$ is an acetyl group.

4. A process as claimed in claims 1 to 3, characterized in that the reaction with sulfur trioxide is carried out at temperatures of 20 to 50 °C with a mixture of $SO_3$ and air or inert gas containing 1 to 10% by volume $SO_3$.

5. A process as claimed in claims 1 to 4, characterized in that $R^1$ is an oleyl group or a fatty alkyl radical consisting predominantly of oleyl groups.

6. The surface-active hydroxysulfonates and alkali metal, alkaline-earth metal and ammonium salts thereof obtained by the process claimed in claims 1 to 5.

7. A mixture of surface-active hydroxysulfonates consisting completely or predominantly of compounds corresponding to formula (II) or (III)

(II) 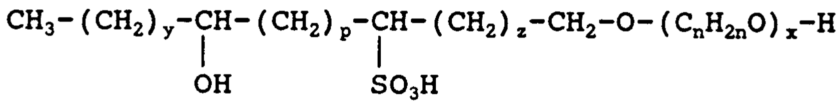

(III) 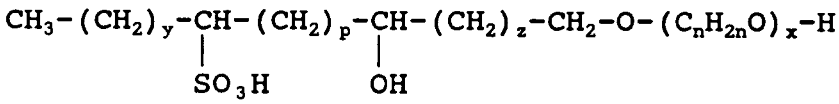

in which y and Z = 0 or numbers of 1 to 18, p = 0, 1 or 2 and the sum of (y + z + p) is a number of 12 to 18, x = 0 or a number of up to 30 and n is an integer of 2 to 4, or of alkali metal, alkaline-earth metal or ammonium salts thereof.

**8.** The use of aqueous solutions of the alkali metal, alkaline-earth metal and ammonium salts of surface-active hydroxysulfonates claimed in claim 6 or 7 for the production of detergents and cleaning preparations.

## Revendications

**1.** Procédé pour la préparation de solutions aqueuses de sels alcalins, alcalins-terreux ou d'ammonium d'hydroxysulfonates tensioactifs, caractérisé en ce que l'on fait réagir avec de l'anhydride sulfurique un ester d'alkyle gras insaturé ou un ester polyoxyalkylique d'alkyle gras insaturé de formule (I) :

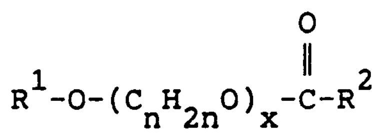

dans laquelle $R^1$ représente un groupe alcényle linéaire ayant de 16 à 22 atomes de carbone ou un groupe alkyle gras essentiellement constitué de groupes oléyle, palmitoléyle, linoléyle, gadoléyle et/ou érucyle, n est un nombre entier allant de 2 à 4, x = 0 ou un nombre allant jusqu'à 30 et $R^2CO$ représente un groupe acyle ayant de 1 à 4 atomes de carbone, on introduit le produit de réaction dans la solution aqueuse de 1 à 2,5 moles d'hydroxyde alcalin, alcalino-terreux ou d'ammonium par mode de $SO_3$ fixé, et on chauffe la solution jusqu'à l'hydrolyse des groupes ester et sultone contenus.

**2.** Procédé selon la revendication 1, caractérisé en ce que n = 2 et x = 0 ou un nombre entier allant jusqu'à 10.

**3.** Procédé selon les revendications 1 et 2, caractérisé en ce que $R^2CO$ est le groupe acétyle.

**4.** Procédé selon les revendications 1 à 3, caractérisé en ce que la réaction avec l'anhydride sulfurique est effectuée à des températures de 20 à 50 ° C, avec un mélange de $SO_3$ et d'air ou de gaz inerte ayant une teneur en $SO_3$ de 1 à 10 % en volume.

**5.** Procédé selon les revendications 1 à 4, caractérisé en ce que $R^1$ est un groupe oléyle ou un radical alkyle gras essentiellement constitué de groupes oléyle.

**6.** Hydroxysulfonates tensioactifs et leurs sels alcalins, alcalino-terreux et d'ammonium, tels qu'ils sont obtenus conformément au procédé selon les revendications 1 à 5.

**7.** Mélange d'hydroxysulfonates tensioactifs, qui est constitué, en totalité ou en majeure partie, de composés de formule (II) ou (III) :

(II) 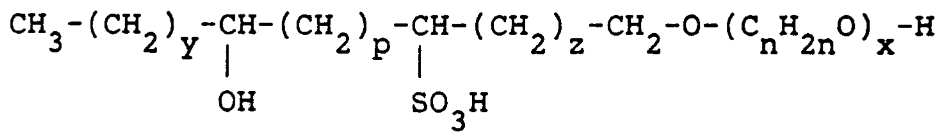

(III) 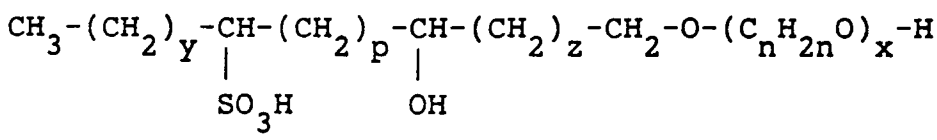

ou de leurs sels alcalins, alcalino-terreux ou d'ammonium, y et z étant 0 ou des nombres allant de 1 à 18, p étant 0, 1 ou 2, et la somme (y + z + p) étant un nombre allant de 12 à 18, et x étant 0 ou un nombre allant jusqu'à 30, et n pouvant être un nombre allant de 2 à 4.

**8.** Utilisation de solutions aqueuses de sels alcalins, alcalino-terreux et d'ammonium d'hydroxysulfonates tensioactifs selon la revendication 6 ou 7, pour la préparation de produits de lavage et de nettoyage.